# EUROPEAN PATENT SPECIFICATION

(11) **EP 2 399 583 B1**
(45) Date of publication and mention of the grant of the patent: **27.01.2016**
(21) Application number: 10743433.4
(22) Date of filing: 16.02.2010
(51) Int. Cl.: A61K 31/655, A61K 31/33, C07C 279/20, C07C 277/02, A61P 5/24, A61P 5/48

(54) **USE OF VAGINALLY-ADMINISTERED INSULIN SENSITIZING AGENTS**
VERWENDUNG VON VAGINAL VERABREICHTEN INSULIN-SENSIBILISIERENDEN MITTELN
UTILISATION D'AGENTS SENSIBILISANTS À L'INSULINE ADMINISTRÉS PAR VOIE VAGINALE

(30) Priority: 18.02.2009 ES 200900448
(43) Date of publication of application: 28.12.2011
(73) Proprietor: ITF Research Pharma, S.L.U., 28108 Alcobendas Madrid (ES)
(72) Inventor: MOSCOSO DEL PRADO, Jaime, 28108 Alcobendas Madrid (ES); BANFI TOSI, Beatriz, 28108 Alcobendas Madrid (ES)
(74) Representative: Linage González, Rafael
(86) International application number: PCT/ES2010/000067
(87) International publication number: WO 2010/094821

(56) References cited:
- WO-A1-03/066061
- WO-A2-95/07697
- WO-A2-03/061362
- MATHUR R ET AL: "Use of metformin in polycystic ovary syndrome", AMERICAN JOURNAL OF OBSTETRICS & GYNECOLOGY, MOSBY, ST LOUIS, MO, US, vol. 199, no. 6, 1 December 2008 (2008-12-01), pages 596-609, XP025779474, ISSN: 0002-9378, DOI: 10.1016/J.AJOG.2008.09.010 [retrieved on 2008-12-01]
- MITKOV M ET AL: "Metformin versus rosiglitazone in the treatment of polycystic ovary syndrome", EUROPEAN JOURNAL OF OBSTETRICS & GYNECOLOGY AND REPRODUCTIVEBIOLOGY, EXCERPTA MEDICA, AMSTERDAM, NL, vol. 126, no. 1, 1 May 2006 (2006-05-01), pages 93-98, XP027921016, ISSN: 0301-2115 [retrieved on 2006-05-01]
- VELAZQUEZ E M ET AL: "Metformin therapy in polycystic ovary syndrome reduces hyperinsulinemia, insulin resistance, hyperandrogenemia, and systolic blood pressure, while facilitating normal menses and pregnancy", METABOLISM, CLINICAL AND EXPERIMENTAL, W.B. SAUNDERS CO., PHILADELPHIA, PA, US, vol. 43, no. 5, 1 May 1994 (1994-05-01), pages 647-654, XP026309040, ISSN: 0026-0495, DOI: 10.1016/0026-0495(94)90209-7 [retrieved on 1994-05-01]
- CATALDO NICHOLAS A ET AL: "Metabolic and ovarian effects of rosiglitazone treatment for 12 weeks in insulin-resistant women with polycystic ovary syndrome.", HUMAN REPRODUCTION (OXFORD, ENGLAND) JAN 2006 LNKD- PUBMED:16155076, vol. 21, no. 1, January 2006 (2006-01), pages 109-120, XP002679114, ISSN: 0268-1161
- ZIAEI S ET AL: "Comparative study and evaluation of side effects of low-dose contraceptive pills administered by the oral and vaginal route.", CONTRACEPTION MAY 2002 LNKD- PUBMED:12057783, vol. 65, no. 5, May 2002 (2002-05), pages 329-331, XP002679115, ISSN: 0010-7824
- BAGIS TAYFUN ET AL: "The effects of short-term medroxyprogesterone acetate and micronized progesterone on glucose metabolism and lipid profiles in patients with polycystic ovary syndrome: a prospective randomized study.", THE JOURNAL OF CLINICAL ENDOCRINOLOGY AND METABOLISM OCT 2002 LNKD- PUBMED:12364431, vol. 87, no. 10, October 2002 (2002-10), pages 4536-4540, XP002679116, ISSN: 0021-972X

## Description

### FIELD OF THE INVENTION

This invention relates to the use of vaginal insulin sensitising agents for the prevention and/or treatment of hyperandrogenism and/or polycystic ovary syndrome and/or related disorders.

### BACKGROUND OF THE INVENTION

In general terms, hyperandrogenism is any clinical or laboratory evidence of an excess of androgens in women. The most frequent clinical evidence of hyperandrogenism in women of childbearing age is hirsutism or acne, with or without anovulation symptoms -such as amenorrhoea or dysfunctional uterine bleeding.

There may be five different causes of hyperandrogenism in women of childbearing potential:
- polycystic ovary syndrome;
- idiopathic hirsutism;
- enzyme deficiencies in the synthesis of steroid hormones;
- androgen-secreting tumours;
- other endocrine disorders such as Cushing's syndrome, etc.

Nevertheless, most women with hyperandrogenism symptoms suffer from polycystic ovary syndrome (80%).

Polycystic ovary syndrome (or PCOS, also called Stein Leventhal syndrome) is the reproductive and hormonal problem that most frequently affects females of reproductive age. It is estimated that approximately 5% of females suffer from this disorder.

According to the American Society for Reproductive Medicine, PCOS is defined by the presence of any two of the following characteristics:
- lack of ovulation over a long period;
- high levels of androgens;
- a large amount of small cysts in the ovaries.

The signs and symptoms of PCOS are related to hyperandrogenism and may include:
- hirsutism, acne and hair loss;
- excess weight or obesity, especially in the waist and abdomen;
- irregular, sporadic or lack of menstrual periods;
- larger and/or polycystic ovaries;
- infertility.

Also, females with PCOS are exposed to a higher risk of developing certain health problems, including:
- metabolic syndrome: disorder with several components, amongst which are type 2 diabetes or insulin resistance, high cholesterol levels, high blood pressure, overweight (especially around waist and abdomen), high levels of C-reactive protein and high levels of blood coagulation factors;
- excessive thickening of the endometrial lining, abundant or irregular bleeding and endometrial cancer.

PCOS therefore has an important effect on the health system and is a matter of concern for the women who suffer from it.

The pharmacological treatment for hyperandrogenism seeks to correct the associated symptoms by reducing androgen serum levels and/or their peripheral action. It must be maintained for a long time since satisfactory clinical effects usually take months to appear.

Certain drugs approved for the treatment of type 2 diabetes, known as insulin-sensitising agents, have proven to be beneficial in patients with PCOS.

Metformin (*N*,*N*-dimethylimidodicarbonimidic diamide, Glucophage®), a biguanide-type insulin-sensitising agent, is available for PCOS treatment (Mathur et al, American Journal of Obstetrics and Ginecology, December 2008, Use of metformin in polycystic ovary syndrome; Velazquez et al, Metabolism, Vol 43, No 5, May 1994, pages 547-654).

Metformin reduces the clinical signs of hyperandrogenism and improves menstrual irregularities. If metformin alone does not restore ovulation, it may improve a woman's response to pharmaceutical products in fertility treatments. The usual oral dose lies between 500 and 2500 mg/day.

Rosiglitazone (Avandia®) and Pioglitazone (Actos®), belonging to the group of thiazolidindione insulin-sensitising agents, are also available in the United States for women with PCOS (Mitkov et al, Eur. Jour. of Obstetrics and Ginecology and Repr. Biol. 126 (2006), 93-98; Cataldo et al, Human Reproduction Vol 21, No 1, pp 109-120, 2006). Thiazolidindiones have demonstrated reducing hyperandrogenism and restoring ovulation in some patients. The recommended oral dose is between 4 to 8 mg/day for rosiglitazone and between 15 and 30 mg/day for pioglitazone.

### SUMMARY OF THE INVENTION

However, insulin-sensitising agents may cause several adverse effects when administered orally, some of which are very serious.

In the case of metformin, the most common, which may cause abandoning the treatment, is gastrointestinal irritation, and occurs in 5 to 20% of cases. Diarrhoea is the most common symptom, followed by vomiting, abdominal pain and a bad taste in the mouth. Lactic acidosis is a rare but serious adverse effect of this medicinal product.

In the case of thiazolidindiones, hepatic toxicity is a rare effect that causes much concern. When using these pharmaceutical products, liver function tests must be performed regularly. And its use is not recommended in patients with evidence of liver disease.

During therapy with these pharmaceutical products there may be fluid retention, which may worsen or trigger congestive heart failure. If you observe a worsening of heart function, you must stop the treatment.

On the other hand, the use of rosiglitazone has been related to a greater risk of events of myocardial ischaemia. Its use is not recommended in patients with ischaemic cardiopathy and/or peripheral arterial disease.

Cases of occurrence or worsening of diabetic macular edema with decreased visual acuity have also been reported during therapy with these pharmaceutical products.

In women, the risk of long-term fractures associated to therapy with thiazolidindiones must also be considered.

The inventors of this invention have found that, surprisingly, the vaginal administration of insulin-sensitising agents allow reaching adequate levels of the medicinal product in the ovaries, obtaining its therapeutic effects while significantly reducing systemic exposure, and therefore their adverse effects. Surprisingly, these agents would produce their anti-androgenic effect without requiring indirect systemic action -via increased sensitivity to insulin- but by direct action on ovarian cells.

In particular, the inventors have observed that the vaginal administration of metformin significantly decreases the plasma levels of testosterone (androgenic hormone). Similarly, they have observed a decrease of atretic follicles (atrophied) in polycystic ovaries.

Therefore, the vaginal administration of insulin-sensitising agents may be useful in the prevention and/or the treatment of PCOS both in patients with high levels of androgens (due to their effect on the plasma concentration of testosterone) and in patients with normal levels of androgens (due to their direct action on the ovarian tissue).

It could also be useful in the prevention and/or treatment of other hyperandrogenic conditions that occur with high plasma levels of androgens, as well as in related disorders, such as hirsutism, acne and/or hair loss.

The first aspect of this invention therefore relates to insulin-sensitising agents for vaginal use in the prevention and/or the treatment of polycystic ovary syndrome and/or hyperandrogenic conditions and/or related disorders.

The second aspect relates to pharmaceutical formulations for vaginal administration containing at least one insulin-sensitising agent for use in the prevention and/or treatment of polycystic ovary syndrome and/or hyperandrogenic conditions and/or related disorders.

### BRIEF DESCRIPTION OF THE DRAWINGS

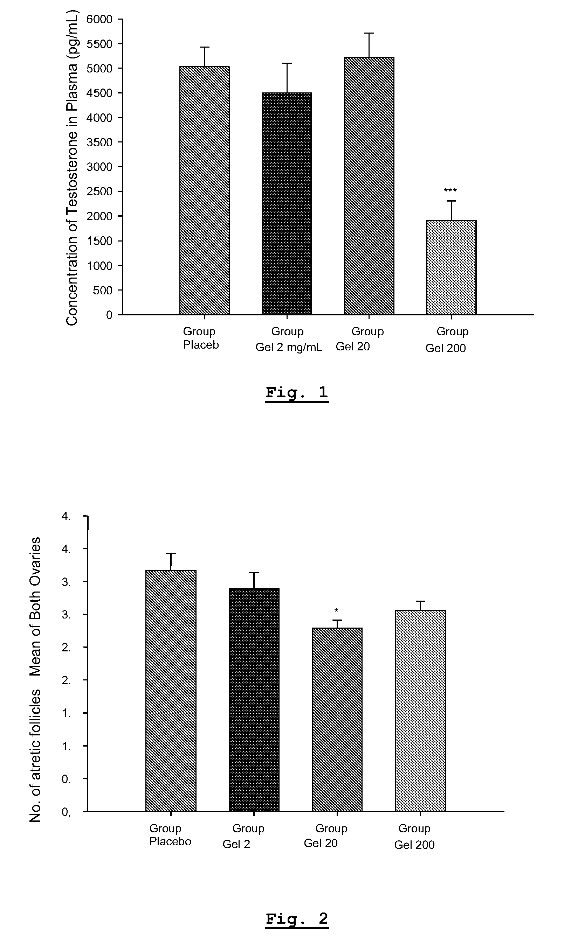

Figure 1: Concentration of testosterone in plasma.
Figure 2: Mean histological assessment of both ovaries (no.
of atretic follicles).

### DETAILED DESCRIPTION OF THE INVENTION

In a particular embodiment, this invention relates to metformin or thiazolidindione for use in the prevention and/or treatment of polycystic ovary syndrome.

In another particular embodiment, this invention relates to metformin or thiazolidindione for use in the prevention and/or treatment of other hyperandrogenic conditions with high plasma levels of androgens.

This invention relates to said pharmaceutical products for use in the prevention and/or treatment of disorders related with hyperandrogenic conditions, such as hirsutism, acne and/or hair loss.

The formulations of insulin-sensitising agent of this invention can be presented in any dosage form suitable for vaginal administration, for example, as a solid, a semisolid or a fluid.

These formulations will comprise a therapeutically effective and non-toxic amount of at least one insulin-sensitising agent -or one of its pharmaceutically acceptable salts together with at least one pharmaceutically acceptable excipient. The excipients may be chosen from any of those known by a person skilled in the art and will be suited to the dosage form to be prepared.

The pharmaceutical formulations of this invention may be contained in any device suitable for vaginal administration, for example, a ring or pessary. The materials to be used in the manufacture of the device may be chosen from any of those known by a person skilled in the art that are pharmaceutically acceptable.

The amount of insulin-sensitising agent that must be administered by vaginal route to treat hyperandrogenism and/or PCOS and/or related disorders safely and effectively depends on many factors, including the patient's age and condition, severity of the disease or disorder, frequency of administration of the formulation, etc.

In a particular embodiment, the metformin dose to be administered by vaginal route will be between 0.01 mg/day and 1000 mg/day; preferably between 0.1 mg/day and 100 mg/day; more preferably between 0.5 and 50 mg/day, between 1 to 10 mg/day.

### EXAMPLE

The following test illustrates the invention.

The study objective was to assess the effect of metformin in a model for polycystic ovary syndrome (PCOS) induced by dehydroepiandrosterone (DHEA) in rats.

### Experimental method

The formulations to be tested were:
- Gels with Metformin at a concentration of 2 mg/mL (FT-147), 20 mg/mL (FT-148) and 200 mg/mL (FT-149).
- Gel without Metformin (Placebo; FT-150).

The gels were prepared according to the following formulae:

| | 2 mg/mL gel (FT-147) | 20 mg/mL gel (FT-148) | 200 mg/mL gel (FT-149) |
|---|---|---|---|
| Metformin | 0.1g | 1g | 10 g |
| Sodium methylparaben | 34.3 mg | 34.3 mg | 34.3 mg |
| Citric acid | 1g | 1g | 1 g |
| 40% Sodium hydroxide | q.s. pH=5.5 approx. | q.s. pH=5.5 approx. | q.s. pH=5.5 approx. |
| Natrosol | 0.85 g | 0.85 g | 0.85 g |
| Water | q.s. 50 g | q.s. 50g | q.s. 50 g |

Female 2-3 weeks-old Sprague-Dawley rats were used.

The animals remained in quarantine for 7 days in the same conditions in which the study was performed. They were weighed as they arrived at the laboratory and every day after that, before the administration of the trial substances and DHEA.

They were kept in 255 x 405 x 197 mm polycarbonate cages, with sawdust bedding.

They were distributed into groups of 5 animals in each cage, chosen at random and were housed in controlled conditions of temperature (22±2°C), light period (12/12 hr light/darkness), air pressure, number of renewals and relative humidity (30-70%).

They were supplied a standard maintenance diet for rodents and were allowed ad libitum access to drinking water.

Four experimental groups with 8 animals each were used in the study:
Group A: Control = Placebo + DHEA
Group B: Dose 1 Trial Substance (0.016 mg/animal) + DHEA
Group C: Dose 2 Trial Substance (0.16 mg/animal) + DHEA
Group D: Dose 3 Trial Substance (1.6 mg/animal) + DHEA

Each animal's dose was calculated based on the concentration of each formulation and the administration volume:
Dose 1: formulation of 2 mg/mL with administration of 8 µl/animal = 0.016 mg/animal
Dose 2: formulation of 20 mg/mL with administration of 8 µl/animal = 0.16 mg/animal
Dose 3: formulation of 200 mg/mL with administration of 8 µl/animal = 1.6 mg/animal

The trial substance (doses of 0.016, 0.16 and 1.6 mg/animal) and the control substance were administered intravaginally as an aqueous gel at a volume of 8 µl/animal.

The DHEA solution was prepared in a sesame seed oil prior to administration and was injected subcutaneously in a skin fold in the posterior thoracic area (dorsal) at a volume of 0.2 mL.

Administration of the trial or control substance was started on day 0 at the dose specified in point 7.1.

Also on day 0, and immediately after the administration of the trial or control substance, DHEA was administered to each animal by subcutaneous injection (6 mg/100 g body weight/0.2 mL of sesame oil).

This regimen was repeated from day 0 to day 14 (for a total of 15 days of treatment).

Blood samples were obtained from every animal on day 15 (24 hr after the last treatment) by puncture of the abdominal vena cava (anticoagulated with sodium heparin) under anaesthesia (pentobarbital sodium).

Plasma was obtained from each blood sample by centrifugation at 5000 g for 5 minutes, which was frozen for later assessment (quantitative determinations) of testosterone levels by rat-specific commercial immunoassay kits (EIA).

After drawing the blood each animal was sacrificed by cervical dislocation and both ovaries were extracted. These were weighed and fixed in buffered formalin for subsequent histological assessment (by haematoxylin and eosin staining).

Three histological cuts were studied from each ovary, taken from 3 representative areas of each ovary (beginning, centre and end), determining the number of atretic follicles (atrophic). A follicle is considered atretic when one of the following features is observed: pyknosis of the granule cells, granule cells present in follicular fluid, hypertrophy of thecal cells).

### Results

### A. Concentration of testosterone in plasma.

Testosterone concentrations in the plasma samples from all animals were determined at the end of the study (Day 15). The results are shown in Table 1:

**Table 1. Concentration of testosterone in plasma.**

| **Study FCI/07/05/FT - PLASMA LEVEL OF TESTOSTERONE** | | | | | | | |
|---|---|---|---|---|---|---|---|
| **GROUP A** | | **GROUP B** | | **GROUP C** | | **GROUP D** | |
| **ANIMAL** | **TESTOSTERONE (pg/mL)** | **ANIMAL** | **TESTOSTERONE (pg/mL)** | **ANIMAL** | **TESTOSTERONE (pg/mL)** | **ANIMAL** | **TESTOSTERONE (pg/mL)** |
| **A1** | 3128 | **B1** | 2300 | **C1** | 6072 | **D1** | 2576 |
| **A2** | 6164 | **B2** | 5474 | **C2** | 3450 | **D2** | 2622 |
| **A3** | 4876 | **B3** | 6578 | **C3** | 4462 | **D3** | 2990 |
| **A4** | 3956 | **B4** | 2714 | **C4** | 3818 | **D4** | 736 |
| **A5** | 5244 | **B5** | 6486 | **C5** | 7406 | **D5** | 598 |
| **A6** | 6670 | **B6** | 5336 | **C6** | 4554 | **D6** | 1518 |
| **A7** | 5474 | **B7** | 4048 | **C7** | 5428 | **D7** | 3404 |
| **A6** | 4692 | **B8** | 3036 | **C6** | 6578 | **D6** | 828 |
| **MEAN** | **5025.5** | **MEAN** | **4496.5** | **MEAN** | **5221** | **MEAN** | **1900.000** |

### B. Histological Assessment

Three histological cuts were studied from each ovary, taken from 3 representative areas (beginning, centre and end), determining the number of atretic follicles. The results obtained are summarised in Tables 2 to 5:

**Table 2. Histological assessment of the ovaries of animals in Group A.**

| **STUDY FCI/07/05/FT - HISTOLOGY** | | | | | | | | |
|---|---|---|---|---|---|---|---|---|
| **CROUP A** | | | | | | | | |
| **NO. OF ATRETIC FOLLICLES LEFT OVARY** | | | | **NO. OF ATRETIC FOLLICLES** | | | | **Mean Atretic Follicles Both Ovaries** |
| **ANIMAL** | Cut-off 1 | Cut-off 2 | Cut-off 3 | **ANIMAL** | Cut-off 1 | Cut-off 2 | Cut-off 3 | |
| **A1** | 2 | 3 | 5 | **A1** | 1 | 2 | 3 | 2.67 |
| **A2** | 2 | 4 | 5 | **A2** | 3 | 6 | 7 | 4.50 |
| **A3** | 1 | 7 | 3 | **A3** | 3 | 5 | 5 | 4.00 |
| **A4** | 2 | 2 | 3 | **A4** | 2 | 3 | 3 | 2.50 |
| **A5** | 6 | 2 | 2 | **A5** | 6 | 5 | 4 | 4.17 |
| **A6** | 3 | 3 | 2 | **A6** | 3 | 6 | 5 | 3.67 |
| **A7** | 1 | 5 | 8 | **A7** | 1 | 4 | 6 | 4.17 |
| **A6** | 2 | 3 | 5 | **A8** | 2 | 4 | 6 | 3.67 |
| **Mean Atretic Follicles Both Ovaries** | | | | | | | | **3.67** |

**Table 3. Histological assessment of the ovaries of animals in Group B.**

| **STUDY FCI/07/05/FT - HISTOLOGY** | | | | | | | | |
|---|---|---|---|---|---|---|---|---|
| **GROUP B** | | | | | | | | |
| **No. of Atretic Follicles Left Ovary** | | | | **No. of Atretic Follicles Right Ovary** | | | | **Mean Atretic Follicles Both Ovaries** |
| **ANIMAL** | Cut-off 1 | Cut-off 2 | Cut-off 3 | **ANIMAL** | Cut-off 1 | Cut-off 2 | Cut-off 3 | |
| **B1** | 5 | 4 | 4 | **B1** | 2 | 4 | 6 | 4.17 |
| **B2** | 3 | 6 | 6 | **B2** | 2 | 4 | 2 | 3.83 |
| **B3** | 4 | 5 | 3 | **B3** | 5 | 5 | 5 | 4.50 |
| **B4** | 5 | 4 | 0 | **B4** | 2 | 3 | 4 | 3.00 |
| **B5** | 3 | 4 | 5 | **B5** | 3 | 3 | 2 | 3.33 |
| **B6** | 3 | 3 | 4 | **B6** | 1 | 2 | 4 | 2.83 |
| **B7** | 1 | 3 | 1 | **B7** | 2 | 6 | 4 | 2.83 |
| **B8** | 1 | 1 | 4 | **B8** | 3 | 3 | 4 | 2.67 |
| **Mean Atretic Follicles Both Ovaries** | | | | | | | | 3.40 |

**Table 4. Histological assessment of the ovaries of animals in Group C.**

| **STUDY FCI/07/05/FT - HISTOLOGY** | | | | | | | | |
|---|---|---|---|---|---|---|---|---|
| **GROUP C** | | | | | | | | |
| **No. of Atretic Follicles Left Ovary** | | | | **No. of Atretic Follicles Right Ovary** | | | | **Mean Atretic Follicles Both Ovaries** |
| **ANIMAL** | Cut-off 1 | Cut-off 2 | Cut-off 3 | **ANIMAL** | Cut-off 1 | Cut-off 2 | Cut-off 3 | |
| **C1** | 2 | 2 | 2 | **C1** | 4 | 4 | 3 | 2.83 |
| **C2** | 2 | 4 | 5 | **C2** | 3 | 2 | 2 | 3.00 |
| **C3** | 2 | 2 | 2 | **C3** | 2 | 4 | 4 | 2.67 |
| **C4** | 3 | 3 | 3 | **C4** | 2 | 4 | 1 | 2.67 |
| **C5** | 2 | 2 | 4 | **C5** | 1 | 5 | 4 | 3.00 |
| **C6** | 2 | 2 | 5 | **C6** | 2 | 3 | 2 | 2.67 |
| **C7** | 3 | 3 | 2 | **C7** | 1 | 2 | 2 | 2.17 |
| **C8** | 2 | 5 | 3 | **C8** | 3 | 3 | 4 | 3.33 |
| **Mean Atretic Follicles Both Ovaries** | | | | | | | | **2.79** |

**Table 5. Histological assessment of the ovaries of animals in Group D.**

| **STUDY FCI/07/05/FT - HISTOLOGY** | | | | | | | | |
|---|---|---|---|---|---|---|---|---|
| | | | | | | | | |

| **No. of Atretic Follicles Left Ovary** | | | | **No. of Atretic Follicles Right Ovary** | | | | **Mean Atretic Follicles Both Ovaries** |
|---|---|---|---|---|---|---|---|---|
| **ANIMAL** | Cut-off 1 | Cut-off 2 | Cut-off 3 | **ANIMAL** | Cut-off 1 | Cut-off 2 | Cut-off 3 | |
| **D1** | 2 | 2 | 3 | **D1** | 2 | 5 | 5 | 3.17 |
| **D2** | 2 | 2 | 3 | **D2** | 4 | 5 | 4 | 3.33 |
| **D3** | 4 | 4 | 4 | **D3** | 3 | 3 | 4 | 3.67 |
| **D4** | 2 | 3 | 3 | **D4** | 4 | 5 | 3 | 3.33 |
| **D5** | 2 | 2 | 4 | **D5** | 2 | 1 | 4 | 2.50 |
| **D6** | 2 | 4 | 3 | **D6** | 1 | 2 | 3 | 2.50 |
| | 3 | 3 | 4 | | 2 | 3 | 3 | 3.00 |
| | 2 | 3 | 3 | | 1 | 4 | 5 | 3.00 |
| **Mean Atretic Follicles Both Ovaries** | | | | | | | | **3.00** |

### Conclusions

The results demonstrate a very significant reduction of testosterone plasma levels in the animals treated with the trial substance (metformin) at a high dose versus the group treated with the control substance.

A significant reduction was also observed in the number of atretic follicles in the animals treated with the trial substance (metformin) at an intermediate dose versus the group treated with the control substance.

## Claims

1. The use of at least one insulin-sensitising agent in the preparation of a pharmaceutical formulation for the prevention and/or treatment of hyperandrogenism and/or polycystic ovary syndrome and/or at least one related disorder of the group consisting of: hirsutism, acne, hair loss, excess weight, obesity, irregular, sporadic or lack of menstrual periods, larger and/or polycystic ovaries and infertility; wherein said formulation is administered intravaginally.

2. The use according to claim 1, for the prevention and/or treatment of polycystic ovary syndrome.

3. The use according to claim 1, for the prevention and/or treatment of irregular, sporadic or lack of menstrual periods, larger and/or polycystic ovaries and/or infertility related to polycystic ovary syndrome.

4. The use according to claim 1, for the prevention and/or treatment of hirsutism, acne and/or hair loss related to hyperandrogenic conditions.

5. The use according to any of claims 1 to 4, wherein the insulin-sensitising agent is metformin or one of its pharmaceutically acceptable salts, hydrates, or polymorphs.

6. The use according to claim 5, wherein the metformin dose ranges between 0.01 mg/day and 1000 mg/day.

7. The use according to claim 6, wherein the metformin dose ranges between 0.1 mg/day and 100 mg/day.

8. The use according to claim 7, wherein the metformin dose ranges between 0.5 mg/day and 50 mg/day.

9. The use according to claim 8, wherein the metformin dose ranges between 1 mg/day and 10 mg/day.

10. The use according to any of claims 1 to 4, wherein the insulin-sensitising agent is a thiazolidindione or one of its pharmaceutically acceptable salts, hydrates, or polymorphs.

11. A pharmaceutical formulation comprising at least one insulin-sensitising agent for use in the prevention and/or treatment of hyperandrogenism and/or polycystic ovary syndrome and/or at least one related disorder of the group consisting of: hirsutism, acne, hair loss, excess weight, obesity, irregular, sporadic or lack of menstrual periods, larger and/or polycystic ovaries and infertility; wherein said pharmaceutical formulation is administered intravaginally.

## Patentansprüche

1. Verwendung von mindestens einem Insulinsensibilisierungsmittel zur Herstellung einer pharmazeutischen Formulierung zur Prävention und/oder Behandlung von Hyperandrogenismus und/oder polyzystischem Ovarsyndrom und/oder mindestens einer verwandten Störung aus der Gruppe bestehend aus: Hirsutismus, Akne, Haarausfall, Übergewicht, Adipositas, unregelmäßigen, sporadischen oder fehlenden Menstruationsperioden, vergrößerten und/oder polyzystischen Ovarien und Infertilität, wobei die Formulierung intravaginal verabreicht wird.

2. Verwendung nach Anspruch 1 zur Prävention und/oder Behandlung des polyzystischen Ovarsyndroms.

3. Verwendung nach Anspruch 1 zur Prävention und/oder Behandlung von unregelmäßigen, sporadischen oder fehlenden Menstruationsperioden, vergrößerten und/oder polyzystischen Ovarien und/oder Infertilität im Zusammenhang mit polyzystischem Ovarsyndrom.

4. Verwendung nach Anspruch 1 zur Prävention und/oder Behandlung von Hirsutismus, Akne und/oder Haarausfall im Zusammenhang mit hyperandrogenen Zuständen.

5. Verwendung nach einem der Ansprüche 1 bis 4, wobei das Insulinsensibilisierungsmittel Metformin oder eines seiner pharmazeutisch annehmbaren Salze, Hydrate oder Polymorphe ist.

6. Verwendung nach Anspruch 5, wobei die Metformindosis im Bereich zwischen 0,01 mg/Tag und 1000 mg/Tag liegt.

7. Verwendung nach Anspruch 6, wobei die Metformindosis im Bereich zwischen 0,1 mg/Tag und 100 mg/Tag liegt.

8. Verwendung nach Anspruch 7, wobei die Metformindosis im Bereich zwischen 0,5 mg/Tag und 50 mg/Tag liegt.

9. Verwendung nach Anspruch 8, wobei die Metformindosis im Bereich zwischen 1 mg/Tag und 10 mg/Tag liegt.

10. Verwendung nach einem der Ansprüche 1 bis 4, wobei das Insulinsensibilisierungsmittel Thiazolidindion oder eines seiner pharmazeutisch annehmbaren Salze, Hydrate oder Polymorphe ist.

11. Pharmazeutische Formulierung, umfassend mindestens ein Insulinsensibilisierungsmittel zur Verwendung in der Prävention und/oder Behandlung von Hyperandrogenismus und/oder polyzystischem Ovarsyndrom und/oder mindestens einer verwandten Störung aus der Gruppe bestehend aus: Hirsutismus, Akne, Haarausfall, Übergewicht, Adipositas, unregelmäßigen, sporadischen oder fehlenden Menstruationsperioden, vergrößerten und/oder polyzystischen Ovarien und Infertilität, wobei die pharmazeutische Formulierung intravaginal verabreicht wird.

## Revendications

1. Utilisation d'au moins un agent sensibilisateur à l'insuline dans la préparation d'une formulation pharmaceutique pour la prévention et/ou le traitement de l'hyperandrogénisme et/ou le syndrome de l'ovaire polykystique et/ou au moins un trouble associé du groupe constitué par: l'hirsutisme, l'acné, l'excès de poids, l'obésité, des périodes menstruelles irrégulières, sporadiques, ou manquantes, des ovaires majeurs et/ou polykystiques et l'infertilité; dans laquelle ladite formulation est administrée par voie intravaginale.

2. Utilisation selon la revendication 1, pour la prévention et/ou le traitement du syndrome des ovaires polykystiques.

3. Utilisation selon la revendication 1, pour la prévention et/ou le traitement de périodes menstruelles irrégulières, sporadiques ou manquantes, des ovaires majeurs et/ou polykystiques et/ou de l'infertilité associée au syndrome d'ovaires polykystiques.

4. Utilisation selon la revendication 1, pour la prévention et/ou le traitement de l'hirsutisme, l'acné, et/ou la perte de cheveux associée aux affections hyperandrogéniques.

5. Utilisation selon l'une quelconque des revendications 1 à 4, dans laquelle l'agent sensibilisateur à l'insuline est de la metformine ou un de ses sels, hydrates o polymorphes pharmaceutiquement acceptables.

6. Utilisation selon la revendication 5, dans laquelle la dose de metformine varie entre 0,01 mg/jour y 100 mg/jour.

7. Utilisation selon la revendication 6, dans laquelle la dose de metformine varie entre 0,1 mg/jour y 100 mg/jour.

8. Utilisation selon la revendication 7, dans laquelle la dose de metformine varie entre 0,5 mg/jour y 50 mg/jour.

9. Utilisation selon la revendication 8, dans laquelle la dose de metformine varie entre 1 mg/jour y 10 mg/jour

10. Utilisation selon l'une quelconque des revendications 1 à 4, dans laquelle l'agent sensibilisateur à l'insuline est une thiazolidindione o un de ses sels, hydrates ou polymorphes pharmaceutiquement acceptables.

11. Une formulation pharmaceutique comprenant au moins un agent sensibilisateur à l'insuline pour son utilisation dans la prévention et/ou le traitement de l'hyperandrogénisme et/ou le syndrome de l'ovaire polykystique et/ou au moins un trouble associé du groupe constitué par: l'hirsutisme, l'acné, la perte de cheveux, l'excès de poids, l'obésité, des périodes menstruelles irrégulières, sporadiques, ou manquantes, des ovaires majeurs et/ou polykystiques et l'infertilité; dans laquelle ladite formulation est administrée par voie intravaginale.
